Europäisches Patentamt

(19) European Patent Office

Office européen des brevets

(11) Publication number: **0 143 847**

**A1**

(12) # EUROPEAN PATENT APPLICATION
published in accordance with Art. 158(3) EPC

(21) Application number: **83903577.1**

(22) Date of filing: **15.11.83**

Data of the international application taken as a basis:

(86) International application number:
**PCT/JP83/00413**

(87) International publication number:
**WO84/01893 (24.05.84 84/13)**

(51) Int. Cl.⁴: **A 61 F 1/03**
**A 61 F 1/00**

(30) Priority: **15.11.82 JP 200849/82**

(43) Date of publication of application:
**12.06.85 Bulletin 85/24**

(84) Designated Contracting States:
**CH DE FR GB LI**

(71) Applicant: **YOUGENGAISYA YOUSHINKAIHATSU**
**1285 Oaza Mizuhara Hirokawamachi Yame-gun**
**Fukuoka-ken 834-01(JP)**

(72) Inventor: **HIMENO, Shinkichi**
**13-29 Maedashi 4-chome Higashiku**
**Fukuoka-shi Fukuoka-ken 812(JP)**

(74) Representative: **Daunton, Derek**
**Barlow, Gillett & Percival 94, Market Street**
**Manchester M1 1PJ(GB)**

(54) **INTRAMEDULLARY PLUG.**

(57) An intramedullary plug employed when an artifical joint prosthesis is secured in bone marrow by means of bone cement has a retaining linear member provided on a plug body. Stretching of the linear member makes it possible to prevent the intramedullary plug slipping downward when the bone cement is compressed, thereby enabling the reliable compacting of the bone cement under pressure, and an improvement in the fixing properties of the bone cement after solidification.

EP 0 143 847 A1

./...

Croydon Printing Company Ltd.

Fig. 3

SPECIFICATION

TITLE OF THE INVENTION

INTRAMEDULLARY PLUG

FIELD OF THE INVENTION

This invention concerns an intramedullary plug employed to prevent the leak-out of bone cement for securing the artificial joint prosthesis and also to support the cement when filling the intramedullary cavity with it in operation for replacement of the hip or knee joint with an artificial joint prosthesis.

BACKGROUND OF THE INVENTION

In the conventional way of operation for replacement of the hip joint with an artificial joint prosthesis, the femoral head to be replaced is removed first and an intramedullary plug is then inserted to the required depth in the intramedullary cavity from the inlet made after excision, and polymethyl methacrylate cement (PMMA cement) is filled in the medullary cavity, and, before solidification of the cement, the stem of prosthesis is inserted.

In replacement operation using an artificial joint prosthesis, the prosthesis is secured by means of bone cement. Therefore the cement-filling technique will largely affect the result of operation.

- 1 -

Conventionally, ball-like formed cement is filled in the medullary cavity by pressing with a finger tip. However, the cement-filling requires a highly skillful technique; i.e. when the finger pressure is too strong, the intramedullary plug will be slipped downward and the cement-filling will not be sufficient, and, in contrast, when the pressure is too weak, there will be cavities in the medullary cavity and, thereby, the cement will be of no use as prosthesis securing material. Besides, pressing the cement only lightly will inevitably result in an unsufficient filling and will not assure the best filling that the cement fits thoroughly to the rugged surface of the medullary cavity.

## SUMMARY OF THE INVENTION

The purpose of this invention is to eliminate such problems as above mentioned by using this intramedullary plug as cement supporting material in order to enable to press sufficiently the bone cement and to assure a good contact between the rugged surface of the medullary cavity and the cement and, thereby, a strong fixation of the artificial joint prosthesis.

For this purpose, this intramedullary plug has a retaining linear member.

Consequently, this invention, which permits using the plug body as support for cement, makes it possible to compress the cement under pressure and thereby enables the reliable compacting of the bone cement, which is the most important in replacement operation using an artificial joint prosthesis, without highly skillful technique. In addition, the structure is very simple: only a retaining linear member is provided on a plug body. Even when necessary to replace the artificial joint prosthesis with a new one after operation because of infection or other diseases, the plug body can be easily taken out only by pulling the linear member.

BRIEF DESCRIPTION OF DRAWINGS

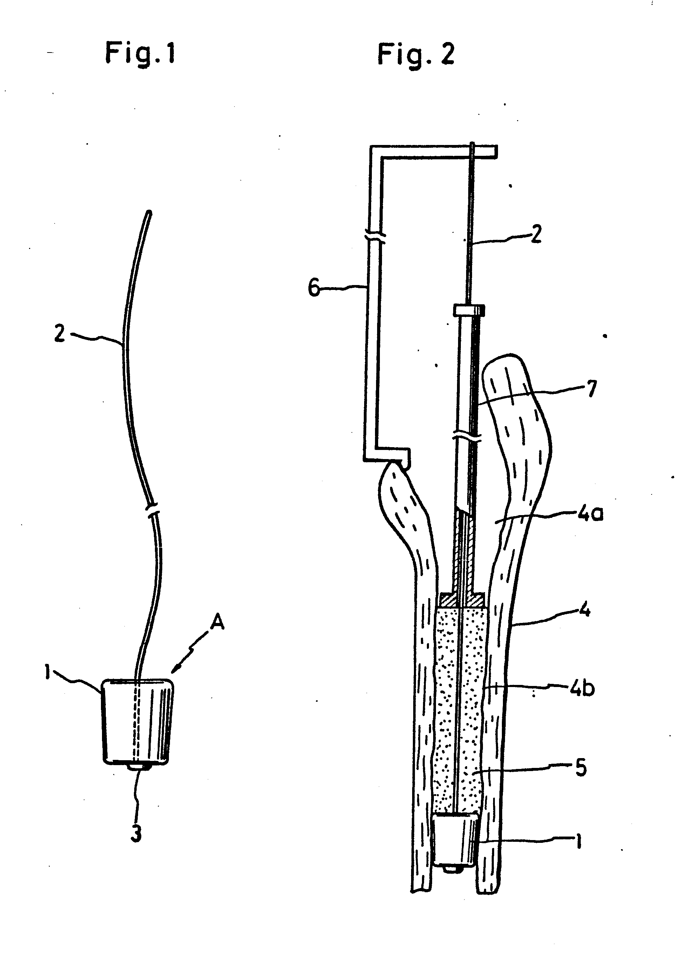

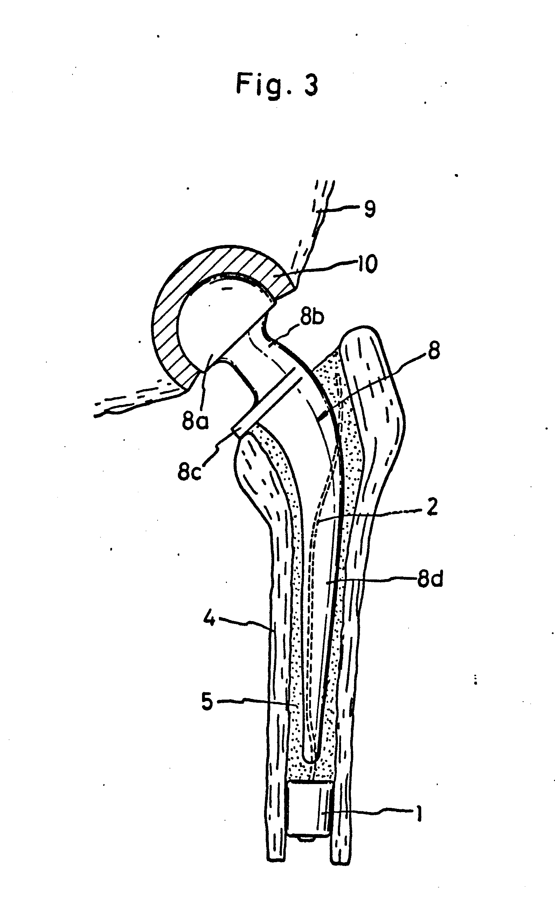

Fig. 1 is an explanatory drawing of the intramedullary plug of this invention. Fig. 2 and Fig. 3 are the side views of the intramedullary plug applied to a living body.

A: Intramedullary plug

1: Plug body

2: Stainless steel wire (retaining linear member)

DETAILED DESCRIPTION OF THE PREFERRED ENBODIMENTS

The preferred embodiments of this invention are explained by referring to drawings, as follows:

Fig. 1 is the front view of the intramedullary plug of the present invention. 1 is the plug body. 2 is a stainless steel wire as retaining linear member. The plug body 1 is made with polyethylene in a taper form with the lower surface with slightly shorter diameter. The lower extremity of the stainless steel wire 2 is connected to a metal fitting 3 fixed to the lower surface of the plug body 1, and prolongs to a certain length (about 50 cm) from the center of the upper surface of the plug body 1.

Fig. 2 and Fig. 3 are explanatory drawings showing the application of the intrameduallary plug A. In Fig. 2, 4 is the femur of which the upper joint part is removed, 5, the bone cement as securing material, 6, a supporting frame used for cement filling and 7, a cement compressor through which the wire 2 is passing. In Fig. 3, 8, an artificial femoral prosthesis which consists of a condyle 8a, neck part 8b, calcar connecting protrusion 8c and stem part 8d, and 9 is the hip bone and 10, an acetabular socket.

The bone cement 5 is compressed in the medullary cavity 4a of the femur 4 using the intramedullary plug A, as follows:

As shown in Fig. 2, place the plug body 1 at the required depth in the medullary cavity 4a and pass the wire 2 through the compressor 7. Fasten up the upper extremity of the wire 2 to the supporting frame 6 which is previously mounted on the upper extremity of the femur 4. Adjust the position of the plug body 1 to the required position by pulling this wire 2. Then, put the cement 5 into the medullary cavity 4a and compact the cement 5 little by little by pressing it with the compressor 7. In this cement filling process, as the pressure force from the compressor 7 is received by the plug body 1, the cement 5 can be compacted under pressure. This action causes the medullary cavity 4a to be filled up with cement 5. Thus, the complete contact between the cement 5 and the rugged internal surface 4b is assured and the even reliable compacting of the bone cement 5 under pressure is possible.

After having filled the medullary cavity with bone cement 5 as mentioned above, the stem part 8d of the artificial femoral joint prosthesis 8 is inserted into the cement 5 before solidification of the cement 5, as shown in Fig. 3. When the prosthesis 8 is inserted into the cement 5, the cement 5 is pressured strongly and, thereby, the prosthesis 8 will be fixed firmly for a long term. The excess of the wire 2 can be cut off.

The load test performed on this invention demonstrated that the yield point was found at an axial load of 100 kg when 5 g of cement was compacted without pressure, whereas the yield point increased to 700 kg when the same amount of cement is compacted under pressure.

The above description explains an example of the practical application of the present invention. However, the concrete composition of the invention is not limited to the above example. For example, any other materials than those above mentioned can be used as components of the plug body, if suitable to the living body, and the shape and structure of the intramedullary plug are not limited to those above mentioned. Namely, those which are used to compose and form intramedullary plug can be employed in this invention, including shape and structure. The flexible material is favorable as linear member, but stiff stuff can be also used as linear member. Any stuffs which are suitable to the living body can be used as linear member, including synthetic resin, metals, etc. The diameter and length of the member can be determined as the occasion demands. And symple-wire, double-wire and twined wire can be used. In addition, the intramedullary plug of this invention can be employed not only in replacement operation using an artificial femoral joint prosthesis 8 shown in the above explanatory drawing, but also in replacement operation using an artificial knee joint prosthesis or for any part which requires a cement-filling.

## Claims

1.  Intramedullary plug characterized by the presence of a linear member which is provided on a plug body.

# Fig.1

# Fig. 2

Fig. 3

0143847

# INTERNATIONAL SEARCH REPORT

0143847

International Application No. PCT/JP83/00413

**I. CLASSIFICATION OF SUBJECT MATTER** (if several classification symbols apply, indicate all) [3]

According to International Patent Classification (IPC) or to both National Classification and IPC

Int. Cl.[3] A61F 1/03, 1/00

**II. FIELDS SEARCHED**

| | Minimum Documentation Searched [4] | |
|---|---|---|
| Classification System | | Classification Symbols |
| I P C | A61F 1/00, 1/03 | |

| | Documentation Searched other than Minimum Documentation to the Extent that such Documents are Included in the Fields Searched [5] | |
|---|---|---|
| | Jitsuyo Shinan Koho | 1926 – 1982 |
| | Kokai Jitsuyo Shinan Koho | 1971 – 1982 |

**III. DOCUMENTS CONSIDERED TO BE RELEVANT [14]**

| Category* | Citation of Document, [16] with indication, where appropriate, of the relevant passages [17] | Relevant to Claim No. [18] |
|---|---|---|
| X | JP,A, 55-146150 (Dow Corning Corp.), 14. November. 1980  (14. 11. 80) & US,A, 4,302,855 | 1 |
| X | GB,A, 2,052,267 (HARDINGE K), 28. January. 1981 (28. 01. 81) | 1 |

* Special categories of cited documents: [15]
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step
"Y" document of particular relevance; the claimed invention cannot be considered to involve an inventive step when the document is combined with one or more other such documents, such combination being obvious to a person skilled in the art
"&" document member of the same patent family

**IV. CERTIFICATION**

| Date of the Actual Completion of the International Search [2] | Date of Mailing of this International Search Report [2] |
|---|---|
| February 1, 1984  (01. 02. 84) | February 13, 1984  (13. 02. 84) |

| International Searching Authority [1] | Signature of Authorized Officer [20] |
|---|---|
| Japanese Patent Office | |

Form PCT/ISA/210 (second sheet) (October 1981)